# EUROPEAN PATENT SPECIFICATION

(11) **EP 4 400 082 B1**
(45) Date of publication and mention of the grant of the patent: **13.11.2024**
(21) Application number: 23151765.7
(22) Date of filing: 16.01.2023
(51) Int. Cl.: A61F 2/40

(54) **INVERSE SHOULDER PROSTHESIS WITH IMPROVED GLENOSPHERE**
INVERSE SCHULTERPROTHESE MIT VERBESSERTER GLENOSPHÄRE
PROTHÈSE D'ÉPAULE INVERSÉE À GLÉNOSPHÈRE AMÉLIORÉE

(43) Date of publication of application: 17.07.2024
(73) Proprietor: Habermeyer, Peter, 80804 München (DE)
(72) Inventor: Habermeyer, Peter, 80804 München (DE)
(74) Representative: Lohr, Jöstingmeier & Partner

(56) References cited:
- EP-A1- 3 178 446
- WO-A1-2018/200127
- US-A1- 2013 261 750
- US-B1- 7 241 314

## Description

### Field of the invention

The invention relates to an inverse shoulder joint prosthesis and in particular to a glenoid implant. The closest prior art is document US 2013/261750 A1, which defines the preamble of claim 1.

### Description of the related art

The shoulder joint is a ball-and-socket-joint. A shoulder joint may be replaced or repaired if it suffers from chronic rotator cuff defect or other maladies, such as arthrosis or fracture. An inverse shoulder prosthesis may be configured to form a fixed center of rotation for the glenohumeral joint in order to restore the mobility of the shoulder if the glenohumeral joint can no longer be centered for pathological reasons. This can be the case with large defects of the rotator cuff. With inverse shoulder prostheses, the original biomechanics are reversed. Hence in contrary to an anatomical shoulder prosthesis, the joint ball, the glenosphere, may be placed on the glenoid side and the artificial prosthesis socket on the humeral head side. Thus, the glenosphere with its spherical joint head and the prosthetic socket may be rotating around the glenosphere, which represents a spherical hinge joint.

WO 2018/200127 A1 discloses arthroplasty implants and methods for orienting joint prostheses.

US8870962B2 discloses an inverse shoulder prosthesis having an adapter anchored in the bone and a spherical hemisphere head, the glenosphere attached to it and a prosthesis socket with polyethylene (PE) inlay embedded in the upper arm shaft. The prosthesis socket and the associated PE inlay have an almost identical radius to the glenosphere. It may be fitting with a spherical glenosphere and identical concavity of the corresponding prosthesis cup (metaphyseal cup). It comprises a pure ball and socket joint that only allows rotation but no translation. Therefore, the patient is limited in the movement in the transverse plane, since the inverse shoulder prosthesis mechanically blocks further movement.

### Summary of the invention

The problem to be solved by the invention is to provide an inverse shoulder prosthesis which can be easily implanted, and which improves the range of motion.

The invention is defined in claim 1. The dependent claims relate to further improvements of the invention.

The inverse shoulder prosthesis has a glenoid implant and a humerus implant. The glenoid implant includes a glenoid body which has a dome shaped articulating surface. A dome has a surface that curves in two directions, and which has a base. The articulating surface may have the shape of a half ellipsoid and preferably of a half three-axial ellipsoid. The base may be circular, it may have an ellipsoid shape, or it may have any other geometrical shape. A dome with a circular base may be known as circular dome. An embodiment relates to a non-circular dome. Opposing to the articulating surface the glenoid implant may include an attachment section. The attachment section may have protrusion which may further have a conical shape which may have a larger size oriented to the base of the dome. The attachment section may be one part with the dome or a separate part. An adapter may fit to the attachment section and may be configured to be mounted to a bone. The adapter may be cup shaped. It may further include a material with good ingrowth properties.

The articulating surface may define a first right hand Cartesian coordinate system with a first x-axis, a first y-axis, a first z-axis and a first point of origin. The first coordinate system may be a Cartesian coordinate system. The point of origin may be the point of the coordinate system where the axes of the coordinate system intersect. The articulating surface extends into the directions of the first x-axis, the first y-axis, and the direction of the first z-axis. The first z-axis may be the center axis of the glenoid body. With respect to a human body, the z-axis may be oriented towards the humerus, while the x-axis may be oriented in an anterior-posterior direction. The y-axis may be oriented in a lateral-medial direction. The articulating surface extends into the positive direction of the first z-axis, in positive and negative directions of the first x-axis, and in positive and negative directions of the first y-axis.

The glenoid body may have a half-spherical cross-sectional area in a plane defined by the first y-axis and the first z-axis. With respect to the human body, this plane may be the sagittal. The glenoid body may have a half-ellipsoidal cross-sectional area in a plane defined by the first x-axis and the first z-axis. With respect to the human body, this plane may be the frontal plane. The base of the dome may be located in a plane defined by the first x-axis and the first y-axis. With respect to the human body, this plane may be the transversal plane. The base of the dome may be largest along the y-axis.

The articulating surface may have at least one first radius located in a plane defined by the first z-axis and the first y-axis. The at least one first radius may be defined by the distance from the first point of origin to the articulating surface. In other words, the at least one first radius may be defined by the length of a straight line starting from the point of origin to the piercing point of the first articulating surface. An intersecting point may be the intersection of a straight line with a surface. The first z-axis and the first radius may form a first angle. Said first angle may be largest if z = 0 and smallest when y = 0. Z = 0 may be the extension of the second radius along the y- axis. The first angle may be 90° when y = 0 and 0° when z = 0. The first angle may have a range of 180°. The first angle may have a range of at least 160°. The first angle may be 90° when y = 0 and 0° or 180° when z = 0. The first angle may be 90° when y =0 and 0° or at least 160° when z = 0. The at least one first radius may be constant. The distance from the point of origin to the articulating surface may stay the same in the plane defined by the first z-axis and the first y-axis.

The articulating surface may have at least one second radius located in a plane defined by the first z-axis and the first x-axis. The at least one second radius may be defined by the distance from the first point of origin to the articulating surface. The at least one second radius may be defined by the length of a straight line starting from the point of origin to the piercing point of the first articulating surface in a plane defined by the first z-axis and the first x-axis. The first z-axis and the at least one second radius may form a second angle. The second angle of the at least one second radius may be smallest if z = 0 and largest when x = 0. Z = 0 may be the extension of the second radius along the x- axis. Said angle may be 90° when x=0 and 0° when z = 0. The second angle may have a range of 180°. The second angle may have a range of at least 160°. The second angle may be 90° when x = 0 and 0° or 180° when z = 0. The second angle may be 90° when x = 0 and 0° or at least 160° when z = 0. The at least one second radius may increase when the second angle approaches 90°. If the second angle is 90° the at least one second radius may be largest. If the second angle is 90° the at least one second radius may correspond to the at least one first radius. If the second angle is 90° the at least one second radius may correspond to extension of the glenoid body along the first z-axis. The distance from the point of origin to the articulating surface may increase along the articulating surface starting from the first x-axis towards the first z-axis.

The second radius at an angle of 0° and 180° or 0° and at least 160° may be smaller than the first radius at an angle of 0° and 180° or and at least 160°. When z = 0, the at least one second radius may be smaller than the at least one first radius. The extension of the at least one first radius along the x-axis may be smaller than the extension of the at least one second radius along the y-axis. In the direction of the first x-axis the extension of the at least one second radius may be in a range of 0.8 to 0.95 of the extension of the at least one first radius. The at least one second radius may be in a range of 0.8 to 0.95 times of the at least one first radius. In the direction of the first x-axis the extension of the second radius may be in a range of 0.9 - 0.92 of the extension of the first radius. The at least one second radius may be in a range of 0.9 to 0.92 times of the at least one first radius. Therefore, the extension of the glenoid body may be largest along the first y-axis and the extension along the first x-axis may be smaller than the extension along the first y-axis. The at least one first radius may correspond the extension of the glenoid body along the first z-axis. In other words, the at least one first radius and the extension of the glenoid body along the first z-axis may be the same. Since the articulating surface is the surface of the glenoid body, the at least one first radius and the at least one second radius of the articulating surface may be the first radius and second radius of the glenoid body.

The humerus implant has a humeral body and an inlay having a concave shape. The inlay having a concave shape may also be referred to as concave inlay. The humeral body may further include a prosthesis stem and/or a prosthesis cup. The inlay may be located inside the prosthesis cup. The inlay may be connected to the prosthesis cup by a loose or fixed connection. The prosthesis cup may be connected to the prosthesis stem by a loose or a fixed connection. The prosthesis cup and the prosthesis stem may be one part or separate parts. The concave inlay may have a second articulating surface, which may include a low friction material. It may include at least one of a polyethylene, polytetrafluorethylene. The concave inlay has a second coordinate system with a second x-axis, a second y-axis, a second z-axis and a second point of origin. The second coordinate system may be a Cartesian coordinate system. The concave inlay may extend into the directions of the second x-axis, the second y-axis, and the second z-axis. With respect to a human body, z-axis may be oriented towards the glenoid of a shoulder, while the x-axis may be oriented in an anterior-posterior direction. The y-axis may be oriented in a craniocaudally direction. The second z-axis may be the center axis of the inlay. The inlay may extend into a negative direction of the second z-axis. The inlay includes at least one inner radius. The center of origin of the at least one inner radius may be the point of origin of the second coordinate system. The at least one inner radius may extend from the second point of origin to the second articulating surface of the inlay. The inlay may have at least an inner first radius and a second inner radius. The first inner radius may be located in a plane defined by the second x-axis and the second z-axis. The second inner radius may be located in a plane defined by the second y-axis and the second z-axis. The second inner radius may be smaller than the first inner radius.

The glenoid body may be placed in the inlay. The glenoid implant may contact the humerus implant. The glenoid body may contact the inlay of the humerus implant. The first articulating surface may contact the second articulating surface. The convex glenoid body and the concave inlay may form a bearing. The first articulating surface and the second articulating surface may form a bearing. The humerus implant and the glenoid implant may move relative to each other. It may be a constrained joint in the x-axis (coronal plane)

The at least one second radius may be smaller than the at least one inner radius of the inlay. The at least one second radius may be smaller than the inner radii of the inlay. This may result in a mismatch of the radii of the articulating surface and the inlay in the x-z plane and hence may result in a gap between the first articulating surface and the second articulating surface in the x-z plane. The at least one second radius may be 0.5 -7.5mm smaller than the inner radius, preferably the at least one second radius may be 2.5 - 7.5mm smaller than the inner radius. This may result in a mismatch of the radii may in a range of 0.5 - 7.5mm, preferably in a range of 2.5 - 7.5 mm. There may be no mismatch of the first radius and the inner radius in the y-z plane. In an embodiment, the articulating surface may have a second radius of 19,5 mm in the z-x plane and the inlay may have a 20 mm radius which may result in a semi-constrained joint in the y-axis (sagittal plane). The mismatch may enable a translational movement of the humerus implant along the x-axes, resulting in a roll and glide movement of the inverse shoulder prosthesis. This mismatch may further enable an anatomically correct external and internal rotation of the inverse shoulder prosthesis. Furthermore, the mismatch may improve the axial rotation of the humerus implant. Due to the morphology of the glenoid body, a combined roll-slide mechanism may occur in the glenohumeral joint during rotation, while a roll-mechanism may occur during arm elevation due to the spherical shape of the glenoid body in the y-z plane.

The glenoid body may be made of metal. The metal may be an alloy. The metal may be stainless steel, a chrome-cobalt alloy, a titanium-based alloy or a nickel-titanium allay. Of course, any other material suitable for a glenoid implant may be also possible. The inlay may be made of plastic, such as polyethylene or polytetrafluorethylene. The glenoid body and the inlay may form a metal-polyethylene bearing coupling. The choice of material can also be changed so that the glenoid component can be made of plastic (and its derivatives) and the humeral inlay can be made of metal or its derivatives.

### Description of Drawings

In the following the invention will be described by way of example, without limitation of the general inventive concept, on examples of embodiment with reference to the drawings.
Figure 1 shows a cross sectional view of an embodiment of the glenoid implant in the y₁-z₁ plane.
Figure 2 shows a cross sectional view of an embodiment of the glenoid implant in the x₁-z₁ plane.
Figure 3 shows a cross sectional view of an embodiment of a humerus implant.
Figure 4 shows a cross sectional view of another embodiment of a humerus implant.
Figure 5 shows a cross sectional view of embodiment of an inverse shoulder prosthesis in the y₁-z₁ (frontal plane).
Figure 6 shows a cross sectional view in the x₁-z₁ plane (transversal plane) of another embodiment of an inverse shoulder prosthesis.
Figure 7 shows a schematic view of a schematic view of a cross sectional view of an embodiment of an inverse shoulder prosthesis.

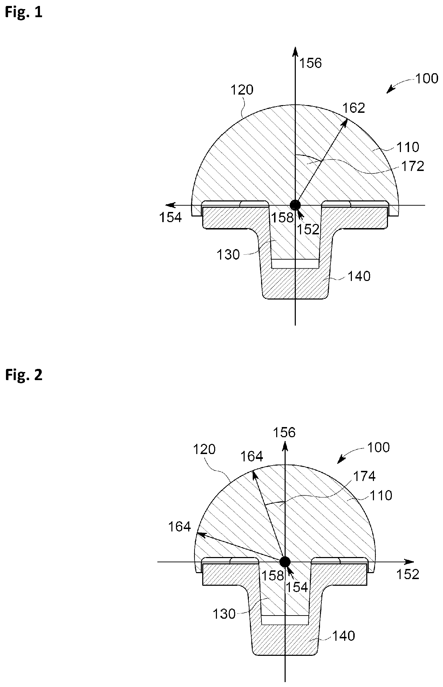

In figure 1 a cross sectional view of an embodiment of a glenoid implant 100 is shown. Figure 1 shows a view of a glenoid implant 100 in the y₁-z₁ plane of an embodiment. The glenoid implant 100 may include a glenoid body 110, a first articulating surface 120, an attachment section 130 and an adapter 140. The glenoid body 110 may be convex. The first articulating surface 120 may be convex and dome shaped. The first articulating surface may have a first coordinate system (x₁,y₁,z₁). The first coordinate system may have a first x-axis x₁ 152, a first y-axis y₁ 154, a first z-axis z₁ 156 and a first point of origin 158. The first articulating surface 120 may extend into the directions of the first x-axis x₁ 152, the first y-axis y₁ 154, and the first z-axis z₁ 156. The extension of the articulating surface 120 and therefore of the glenoid body 110 along the first y-axis y₁ 154 may be greater than the extension of the articulating surface 120 and therefore of the glenoid body 110 along the first x-axis x₁ 152. The glenoid body may be convex and may extend along the positive direction of the first z-axis z₁ 156. The first z-axis z₁ 156 may the center axis of the glenoid body 110. The first z-axis z₁ 156 may the center axis of the articulating surface 120. The first z-axis z₁ 156 may the center axis of the glenoid implant 100. The articulating surface 120 may have at least one first radius 162. The at least one first radius 162 may not vary in the z₁ - y₁ plane. A first angle 172 may be located between the first z-axis z₁ 156 and the least one first radius 162. The first angle 172 may be 90° when z₁ 156 = 0 and 0° when y₁ 154 = 0. The first angle 172 may have a range of 180°. The first angle 172 may have a range of at least 160°. The first angle 172 may be 90° when y₁ 154 = 0 and 0° or 180° when z₁ 156 = 0. The first angle 172 may be 90° when y₁ 154 = 0 and 0° or at least 160° when z₁ 156 = 0. The glenoid body 110 may have a half spherical shape in the z₁- y₁ plane. The first articulating surface 120 may be the outer surface of the glenoid body 110. The glenoid implant 100 may have an attachment section 130 in the negative direction of the z-axis z₁ 156. The attachment section 130 may be connected to the glenoid body 110. Opposing to the first articulating surface 120 may be an attachment section 130 and an adapter 140. The attachment section 130 may be in a recess, for example a conical recess, adapted to hold the attachment section 130. The attachment section 130 may be connected to the glenoid body 110 by a loose or a fixed connection. The attachment section and the glenoid body may be monolithic, the attachment section may be part of the glenoid body. The adapter 140 may be connected to the glenoid body 110 opposing to the first articulating surface 120. The adapter 140 may be connected to the glenoid body 110 by a loose or a fixed connection.

Figure 2 shows a cross sectional view of the glenoid implant 100 of the embodiment of figure 1 in the x₁-z₁ plane. The glenoid implant 100 may include a glenoid body 110, a first articulating surface 120, an attachment section 130 and an adapter 140. The first articulating surface 120may have at least one second radius 164 in the x₁-z₁ plane. A second angle 174 may be located between the first z-axis z₁ 156 and the least one second radius 164. The second angle 174 may be 90° when z₁ = 0 and 0° when x₁ 152 = 0. The second angle 174 may have a range of 180°. The second angle 174 may have a range of at least 160°. The second angle 174 may be 90° when x₁ 152 = 0 and 0° or 180° when z₁ = 0. The second angle 174 may be 90° when x₁ 152 0 and 0° or at least 160° when z₁ = 0.The at least one second radius 164 may vary depending on the second angle 174. The at least one second radius 164 may be smallest, when the second angle 174 is 0° (164₁) and may be largest when the second angle 174 is 90° (164ₙ). When 174 is 90°, the at least one second radius 164 may correspond to the at least one first radius 162. In other words, when 174 is 90° the at least one second radius 164 and the at least one first radius 162 may be the same. The first articulating surface 120 may have the shape of a half ellipsoid and preferably of a half three-axial ellipsoid in the z₁ - x₁ plane. The glenoid body 110 may have a half-ellipsoid, preferably of a half three-axial ellipsoid, cross sectional area in a plane defined by the first x-axis and the first z-axis.

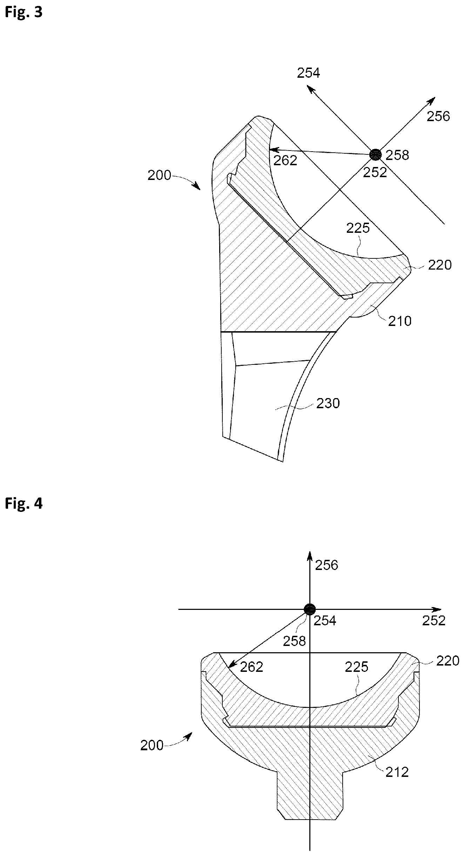

In figure 3 a cross sectional view of an embodiment of a humerus implant 200 is shown. Figure 3 shows a view of a humerus implant 200 in the y₂-z₂ plane of an embodiment. The humerus implant 200 may include prosthesis cup 210, an inlay 220 and a prosthesis stem 230. The inlay 220 may have a second articulating surface 225. The inlay 220 may have a concave shape. The inlay 220 may be located inside the prosthesis cup 210. The inlay 220 may be connected to the prosthesis cup 210 be a loose or fixed connection. The prosthesis cup 210 may be connected to the prosthesis stem 230. The prosthesis cup 210 may be connected to the prosthesis stem 230 by a loose or a fixed connection. The prosthesis cup 210 and the prosthesis stem 230 may be monolithic. The inlay 220 may have a second coordinate system (x₂, y₂, z₂) with a second x-axis x₂ 252, a second y-axis y₂ 254, a second z-axis z₂ 256 and a second point of origin 258. The inlay 220 may extend into the directions of the second x-axis x₂ 252, the second y-axis y₂ 254, and the second z-axis z₂ 256. The second z-axis z₂ 256 may be the center axis of the inlay 220. The inlay 220 may extend into the negative direction of the second z-axis z₂ 256. The inlay 220 may have a second articulating surface 225. The inlay 220 may have at least one inner radius 262. The center of origin of the at least one inner radius 262 may be the point of origin of the second coordinate system. The at least one inner radius 262 may extend from the second point of origin to the second articulating surface 225. The inlay 220 may have at least a first inner radius and a second inner radius. The first inner radius may be located in a plane defined by the second x-axis x₂ 252 and the second z-axis z₂ 256. The second inner radius may be located in a plane defined by the second y-axis y1 154 and the second z-axis. The second inner radius may be smaller than the first inner radius.

In figure 4 a cross sectional view of another embodiment of a humerus implant 200 is shown. Figure 4 shows a view of a humerus implant 200 in the x₂-z₂ plane. The humerus implant 200 may include prosthesis cup 212 and an inlay 220. The prosthesis cup 212 may be connected to prosthesis stem (not shown). The inlay 220 may have a second articulating surface 225. The inlay of this embodiment may correspond to the inlay of the embodiment of Figure 3 in the x₂-z₂ plane.

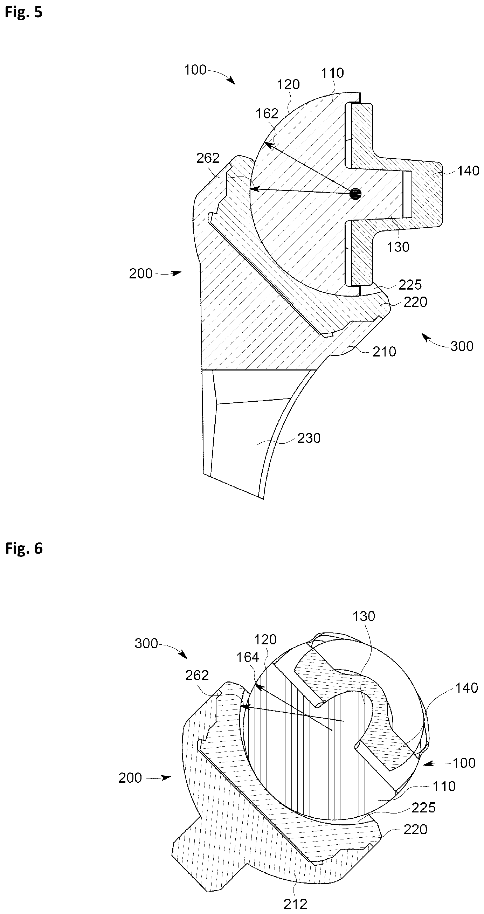

Figure 5 shows a cross sectional view of embodiment of an inverse shoulder prosthesis 300 in the y₁-z₁ (frontal plane). The inverse shoulder prosthesis 300 may include a glenoid implant 100 and a humerus implant 200. The glenoid implant 100 may include a glenoid body 110, a first articulating surface 120, an attachment section 130, at least one first radius 162, an adapter 140. The humerus implant 200 may include a prosthesis cup 210, an inlay 220, a second articulating surface 225, at least one inner radius 262 and a prosthesis stem 230. The glenoid body may have a half spherical cross section. The at least one first radius 162 may be a spherical radius. The at least one first radius 162 may match the inner radius 262. The first articulating surface 120 and the second articulating surface 225 may form a bearing. The matching radii may accomplish an angular rolling-movement in the y₁-z₁ plane. In an embodiment, the inner radius 262 may be 19.7 mm and the at least one first radius 162 may be 19.5 mm. The y₁-z₁ plane may be the frontal plane of the inverse shoulder prosthesis 300.

In figure 6 a cross sectional view in the x₁-z₁ plane (transversal plane) of another embodiment of an inverse shoulder prosthesis 300 is shown. The inverse shoulder prosthesis 300 may include a glenoid implant 100 and a humerus implant 200. The glenoid implant 100 may include a glenoid body 110, a first articulating surface 120, an attachment section 130, at least one second radius 164, an adapter 140. The humerus implant 200 may include a prosthesis cup 210, an inlay 220, a second articulating surface 225, at least one inner radius 262 and a prosthesis stem 230 (not shown). The at least one second radius 164 may be smaller than the at least one inner radius 262. There may be a mismatch between the at least one second radius 164 and the at least one inner radius 262 The mismatch may be in a range of 2.5 mm - 7.5 mm. The mismatch may be in the transversal plane of the inverse shoulder prosthesis 300. This mismatch may allow an oscillation in the transversal plane. This oscillation may be a translational movement. The mismatch may enable a translational movement of the humerus implant 200 along the x-axes, resulting in a roll and glide movement of the inverse shoulder prosthesis 300. This mismatch may further enable an anatomically correct external and internal rotation of the inverse shoulder prosthesis 300. Furthermore, the mismatch may improve the axial rotation of the humerus implant 200. Due to the morphology of the glenoid body 110, a combined roll-slide mechanism may occur in the glenohumeral joint during rotation, while a roll-mechanism may occur during arm elevation due to the spherical shape of the glenoid body 110 in the y-z plane. In an embodiment, the inner radius 262 may be 19.7 mm and the at least one second radius 162 may be 17 mm.

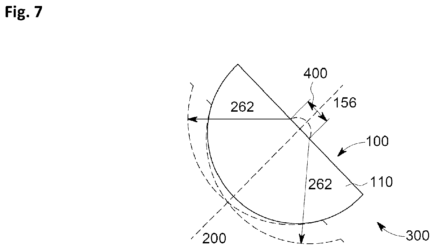

Figure 7 shows a schematic view of a schematic view of a cross sectional view of an embodiment of an inverse shoulder prosthesis 300. Figure 7 shows the possible oscillation curve of the humerus implant 200. Due to the mismatch 400 between the radii of the inlay 220 and the first articulating surface 120, there may be a roll and glide movement of the joint. Due to this roll and glide movement, a greater range of motion of the prosthesis may be accomplished.

### List of reference numerals

- 100: glenoid implant
- 110: glenoid body
- 120: first articulating surface
- 130: attachment section
- 140: adapter
- 152: first x-axis / x₁
- 154: first y-axis / y₁
- 156: first z-axis / z₁
- 158: first point of origin
- 162: first radius
- 164: second radius
- 172: first angle
- 174: second angle
- 200: humerus implant
- 210: humeral body
- 212: prosthesis cup
- 220: inlay
- 225: second articulating surface
- 230: prosthesis stem
- 252: second x-axis / x₂
- 254: second y-axis / y₂
- 256: second z-axis / z₂
- 258: second point of origin
- 262: inlay radius
- 300: inverse shoulder prosthesis
- 400: Mismatch

## Claims

1. An inverse shoulder prosthesis (300) comprising a glenoid implant (100) and a humerus implant (200),
the glenoid implant (100) having a glenoid body (110), with an articulating surface (120) having a convex dome shape,
the articulating surface (120) defining a first right hand coordinate system having a first x-axis (152), a first y-axis (154), a first z-axis (156) and a first point of origin (158), the first z-axis (156) being a center axis of the glenoid body (110),
the articulating surface (120) extending into a positive direction of the first z-axis (156), in positive and negative directions of the first x-axis (152), and in positive and negative directions of the first y-axis (154), or the articulating surface (120) oriented towards a humerus and the articulating surface (120) having a base in an anterior-posterior and a lateral-medial direction, the articulating surface (120) having a first radius (162) in a plane defined by the first y-axis (154) and the first z-axis (156),and a second radius (164) in a plane defined by the first x-axis (152) and the first z-axis (156), or the articulating surface (120) having a first radius (162) in a sagittal plane, and a second radius (164) in a frontal plane, and
the humerus implant (200) having a humeral body (210, 212) and an inlay (220), having a concave shape,
the inlay (220) having a second coordinate system having a second x-axis (252), a second y-axis (254), a second z-axis (256) and a second point of origin (258),
the second z-axis (256) being the center axis of the inlay (220),
the inlay (220) extending into a negative direction of the second z-axis (256), or the concave inlay (220) having a center axis oriented towards a glenoid of a shoulder, and the inlay (220) having a concave shape and a base in an anterior-posterior direction and a craniocaudally direction, the inlay (220) having an inner radius(262),
**characterized in that**
the second radius (164) is in a range of 0.8 to 0.95 times of the first radius (162) and the first radius (164) is smaller than the inner radius (262) of the inlay (220).

2. An inverse shoulder prosthesis according to claim 1, wherein the second radius (164) is in a range of 0.9 to 0.92 times of the first radius (162).

3. An inverse shoulder prosthesis according to any of the preceding claims, wherein the first radius (162) and an extension of the glenoid body (110) along the first z-axis (156) are the same.

4. An inverse shoulder prosthesis according to any of the preceding claims, the glenoid implant (110) being displaceable along the first x-axis (152) within the inlay (220).

5. An inverse shoulder prosthesis according to any of the preceding claims, wherein the second radius is 0.5 - 7.5 mm smaller than the inner radius of the inlay (220).

6. An inverse shoulder prosthesis according to any of the preceding claims, wherein the second radius is 2.5 - 7.5 mm smaller than the inner radius of the inlay (220).

7. An inverse shoulder prosthesis according to any of the preceding claims, the first radius (162) being defined by the distance from the first point of origin (158) to the articulating surface (120), wherein the distance from the point of origin to the articulating surface (120) stays the same in the plane defined by the first z-axis (156) and the first y-axis (154).

8. An inverse shoulder prosthesis according to any of the preceding claims, the second radius (164) being defined by the distance from the first point of origin (158) to the articulating surface (120), wherein the distance from the point of origin to the articulating surface (120) increases along the articulating surface starting from the first x-axis (152) towards the first z-axis (156).

9. An inverse shoulder prosthesis according to any of the preceding claims, wherein a first angle (172) is formed between the first z-axis (156) and the first radius (162).

10. An inverse shoulder prosthesis according to any of the preceding claims, wherein a second angle (174) is formed between the first z-axis (156) and the second radius (162), the second angle (174) being 90° when x₁=0 and 0° when z₁ = 0, wherein the second radius increases when the second angle (174) approaches 90°.

11. An inverse shoulder prosthesis according to any of the preceding claims, wherein the inlay has at least one radius around the second z-axis (256).

## Patentansprüche

1. Eine inverse Schulterprothese (300), umfassend ein Glenoidimplantat (100) und ein Humerusimplantat (200),
wobei das Glenoidimplantat (100) einen Glenoidkörper (110), mit einer Gelenkfläche (120) mit einer konvexen Kuppelform aufweist,
wobei die Gelenkfläche (120) ein erstes rechtsseitiges Koordinatensystem mit einer ersten x-Achse (152), einer ersten y-Achse (154), einer ersten z-Achse (156) und einem ersten Ursprungspunkt (158) definiert, wobei die erste z-Achse (156) eine Mittelachse des Glenoidkörpers (110) ist,
wobei sich die Gelenkfläche (120) in eine positive Richtung der ersten z-Achse (156), in positive und negative Richtungen der ersten x-Achse (152), und in positive und negative Richtungen der ersten y-Achse (154) erstreckt, oder die Gelenkfläche (120) in Richtung eines Humerus ausgerichtet ist und die Gelenkfläche (120) eine Basis in einer Anterior-Posterior-Richtung und einer Lateral-Medial-Richtung aufweist, wobei die Gelenkfläche (120) einen ersten Radius (162) in einer Ebene aufweist, die durch die erste y-Achse (154) und die erste z-Achse (156) definiert ist, und einen zweiten Radius (164) in einer Ebene aufweist, die durch die erste x-Achse (152) und die erste z-Achse (156) definiert ist, oder die Gelenkfläche (120) einen ersten Radius (162) in einer Sagittalebene und einen zweiten Radius (164) in einer Frontalebene aufweist, und
wobei das Humerusimplantat (200) einen Humeruskörper (210, 212) und ein Inlay (220), mit einer konkaven Form aufweist,
wobei das Inlay (220) ein zweites Koordinatensystem mit einer zweiten x-Achse (252), einer zweiten y-Achse (254), einer zweiten z-Achse (256) und einem zweiten Ursprungspunkt (258) aufweist,
wobei die zweite z-Achse (256) die Mittelachse des Inlays (220) ist,
wobei sich das Inlay (220) in eine negative Richtung der zweiten z-Achse (256) erstreckt, oder das konkave Inlay (220) eine Mittelachse aufweist, welche in Richtung eines Glenoids einer Schulter ausgerichtet ist, und das Inlay (220) eine konkave Form und eine Basis in einer Anterior-Posterior-Richtung und einer kraniokaudalen Richtung aufweist, wobei das Inlay (220) einen Innenradius (262) aufweist,
**dadurch gekennzeichnet**,
der zweite Radius (164) in einem Bereich vom 0,8- bis 0,95-fachen des ersten Radius (162) liegt und der erste Radius (164) kleiner ist als der Innenradius (262) des Inlays (220).

2. Eine inverse Schulterprothese gemäß Anspruch 1,
wobei der zweite Radius (164) in einem Bereich vom 0,9- bis 0,92-fachen des ersten Radius (162) liegt.

3. Eine inverse Schulterprothese gemäß einem der vorhergehenden Ansprüche,
wobei der erste Radius (162) und eine Erstreckung des Glenoidkörpers (110) entlang der ersten z-Achse (156) gleich sind.

4. Eine inverse Schulterprothese nach einem der vorhergehenden Ansprüche, wobei das Glenoidimplantat (110) entlang der ersten x-Achse (152) innerhalb des Inlays (220) verschiebbar ist.

5. Eine inverse Schulterprothese nach einem der vorhergehenden Ansprüche, wobei der zweite Radius 0,5 - 7,5 mm kleiner ist als der Innenradius des Inlays (220).

6. Eine inverse Schulterprothese nach einem der vorhergehenden Ansprüche, wobei der zweite Radius 2,5 - 7,5 mm kleiner ist als der Innenradius des Inlays (220).

7. Eine inverse Schulterprothese nach einem der vorhergehenden Ansprüche, wobei der erste Radius (162) durch den Abstand vom ersten Ursprungspunkt (158) zur Gelenkfläche (120) definiert ist, wobei der Abstand vom Ursprungspunkt zur Gelenkfläche (120) in der durch die erste z-Achse (156) und die erste y-Achse (154) definierten Ebene gleich bleibt.

8. Eine inverse Schulterprothese nach einem der vorhergehenden Ansprüche, wobei der zweite Radius (164) durch den Abstand vom ersten Ursprungspunkt (158) zur Gelenkfläche (120) definiert ist, wobei der Abstand vom Ursprungspunkt zur Gelenkfläche (120) entlang der Gelenkfläche ausgehend von der ersten x-Achse (152) in Richtung zur ersten z-Achse (156) zunimmt.

9. Eine inverse Schulterprothese nach einem der vorhergehenden Ansprüche, wobei ein erster Winkel (172) zwischen der z-Achse (156) und dem ersten Radius (162) gebildet wird.

10. Eine inverse Schulterprothese nach einem der vorhergehenden Ansprüche, wobei ein zweiter Winkel (174) zwischen der ersten z-Achse (156) und dem zweiten Radius (162) gebildet wird, wobei der zweite Winkel (174) 90° beträgt, wenn x₁ = 0, und 0° beträgt, wenn z₁ = 0, wobei der zweite Radius zunimmt, wenn sich der zweite Winkel (174) 90° nähert.

11. Eine inverse Schulterprothese nach einem der vorhergehenden Ansprüche, wobei das Inlay mindestens einen Radius um die zweite z-Achse (256) herum aufweist.

## Revendications

1. Prothèse d'épaule inversée (300) comprenant un implant glénoïdien (100) et un implant d'humérus (200),
l'implant glénoïdien (100) ayant un corps glénoïdien (110), avec une surface d'articulation (120) ayant une forme de dôme convexe, la surface d'articulation (120) définissant un premier repère direct ayant un premier axe x (152), un premier axe y (154), un premier axe z (156) et un premier point d'origine (158), le premier axe z (156) étant un axe central du corps glénoïdien (110),
la surface d'articulation (120) s'étendant dans une direction positive du premier axe z (156), dans des directions positive et négative du premier axe x (152), et dans des directions positive et négative du premier axe y (154), ou la surface d'articulation (120) étant orientée vers un humérus et la surface d'articulation (120) ayant une base dans une direction antéro-postérieure et une direction latérale-médiale,
la surface d'articulation (120) ayant un premier rayon (162) dans un plan défini par le premier axe y (154) et le premier axe z (156), et un second rayon (164) dans un plan défini par le premier axe x (152) et le premier axe z (156), ou la surface d'articulation (120) ayant un premier rayon (162) dans un plan sagittal, et un second rayon (164) dans un plan frontal, et
l'implant d'humérus (200) ayant un corps huméral (210, 212) et un encastrement (220), ayant une forme concave,
l'encastrement (220) ayant un second repère ayant un second axe x (252), un second axe y (254), un second axe z (256) et un second point d'origine (258),
le second axe z (256) étant l'axe central de l'encastrement (220), l'encastrement (220) s'étendant dans une direction négative du second axe z (256), ou l'encastrement (220) concave ayant un axe central orienté vers une glène d'une épaule, et l'encastrement (220) ayant une forme concave et une base dans une direction antéro-postérieure et une direction crânio-caudale, l'encastrement (220) ayant un rayon interne(262),
**caractérisée en ce que**
le second rayon (164) est dans une plage de 0,8 à 0,95 fois le premier rayon (162) et le premier rayon (164) est plus petit que le rayon interne (262) de l'encastrement (220).

2. Prothèse d'épaule inversée selon la revendication 1, dans laquelle le second rayon (164) est dans une plage de 0,9 à 0,92 fois le premier rayon (162).

3. Prothèse d'épaule inversée selon l'une quelconque des revendications précédentes,
dans laquelle le premier rayon (162) et une extension du corps glénoïdien (110) suivant le premier axe z (156) sont identiques.

4. Prothèse d'épaule inversée selon l'une quelconque des revendications précédentes,
l'implant glénoïdien (110) étant déplaçable suivant le premier axe x (152) au sein de l'encastrement (220).

5. Prothèse d'épaule inversée selon l'une quelconque des revendications précédentes, dans laquelle le second rayon est de 0,5 à 7,5 mm plus petit que le rayon interne de l'encastrement (220).

6. Prothèse d'épaule inversée selon l'une quelconque des revendications précédentes,
dans laquelle le second rayon est de 2,5 à 7,5 mm plus petit que le rayon interne de l'encastrement (220).

7. Prothèse d'épaule inversée selon l'une quelconque des revendications précédentes,
le premier rayon (162) étant défini par la distance entre le premier point d'origine (158) et la surface d'articulation (120), dans laquelle la distance entre le point d'origine et la surface d'articulation (120) reste la même dans le plan défini par le premier axe z (156) et le premier axe y (154).

8. Prothèse d'épaule inversée selon l'une quelconque des revendications précédentes,
le second rayon (164) étant défini par la distance entre le premier point d'origine (158) et la surface d'articulation (120), dans laquelle la distance entre le point d'origine et la surface d'articulation (120) augmente suivant la surface d'articulation à partir du premier axe x (152) vers le premier axe z (156).

9. Prothèse d'épaule inversée selon l'une quelconque des revendications précédentes,
dans laquelle un premier angle (172) est formé entre le premier axe z (156) et le premier rayon (162).

10. Prothèse d'épaule inversée selon l'une quelconque des revendications précédentes, dans laquelle
un second angle (174) est formé entre le premier axe z (156) et le second rayon (162), le second angle (174) étant de 90° lorsque x₁ = 0 et de 0° lorsque zi = 0, dans laquelle le second rayon augmente lorsque le second angle (174) s'approche de 90°.

11. Prothèse d'épaule inversée selon l'une quelconque des revendications précédentes, dans laquelle
l'encastrement présente au moins un rayon autour du second axe z (256).
